# FASCICULE DE BREVET EUROPEEN

(11) **EP 1 021 433 B1**
(45) Date de publication et mention de la délivrance du brevet: **14.11.2001**
(21) Numéro de dépôt: 97933741.7
(22) Date de dépôt: 15.07.1997
(51) Int. Cl.: C07D 319/06, C12P 7/18

(54) **PROCEDE DE PRODUCTION DE DERIVES DE DIOL-1,3 NATURELS ET DE DERIVES DE 1,3-DIOXANE NATURELS CORRESPONDANTS**
VERFAHREN ZUR HERSTULLUNG NATÜRLICHER 1,3 DIOL DERIVATE UND KORRESPONDIERENDE NATÜRLICHE 1,3 DIOXAN DERIVATE
METHOD FOR PRODUCING NATURAL DIOL-1,3 DERIVATIVES AND CORRESPONDING NATURAL 1,3-DIOXANE DERIVATIVES

(30) Priorité: 16.07.1996 FR 9608852
(43) Date de publication de la demande: 26.07.2000
(73) Titulaire: PERNOD-RICARD, 75008 Paris (FR)
(72) Inventeur: BRUNERIE, Pascal, Marc, F-94440 Santeny (FR)
(74) Mandataire: Warcoin, Jacques
(86) Numéro de dépôt international: FR9701309
(87) Numéro de publication internationale: WO9802428

(56) Documents cités:
- JANUSZ KULESZA ET AL.: "Ausnutzung der n-alpha-Olefine C6-C11 bei der Synthese der Riechstoffe II. Teil" RIECHSTOFFE AROMEN KORPERPFLEGEMITTEL, vol. 19, no. 4, April 1969, HANNOVER-BEMRODE DE, page 157 XP000647556
- V. V. KUTZNETSOV ET AL.: "Alkylboronic Acid Ester in Reaction of Olefins with Paraform" RUSSIAN JOURNAL OF GENERAL CHEMISTRY, vol. 64, no. 10, October 1994, NTS BUREAU US, pages 1555-1556, XP002027956
- ADAM SOKOLOWSKI ET AL.: "Acetals and Ethers. 11. Solubility of Alkyl- Substituted 1,3-Dioxolanes and 1,3-Dioxanes in Water" THE JOURNAL OF PHYSICAL CHEMISTRY, vol. 88, no. 4, 16 February 1984, pages 807-809, XP000646189

## Description

La présente invention concerne la production de diols naturels et leurs applications.

La pomme est un produit agricole dont l'importance économique justifie les études qui lui sont consacrées. Celle-ci est, en effet, un fruit de consommation courante pour le marché de bouche, mais également un fruit industriel important pour l'industrie agro-alimentaire car il est à la base de nombreux produits comme les jus de fruits, les préparations de fruits destinées à d'autres industries, les compotes et confitures ou encore des boissons à faible degré alcoolique comme les cidres.

De nombreuses études ont été consacrées à l'arôme de la pomme, qui ont permis d'identifier plus de 350 composés volatils (Berger et al., 1988) parmi lesquels certains ont une contribution importante à l'ensemble des propriétés sensorielles de la pomme. Parmi ceux-ci, les aldéhydes en C6 comme le cis-3-héxénal, le trans-2-héxénal, les alcools correspondants, certains esters comme le butyrate d'éthyle, le 2-méthylbutyrate d'éthyle, ou la β-damascenone, ont un rôle essentiel (Belitz, 1992 ; Dürr et al., 1981).

Au cours de ces études, la structure de deux composés volatils se trouvant en quantité assez importante dans certaines variétés de pomme a été élucidée, il s'agit de deux diols en C8 homologues : l'octanediol-1,3 et le cis-octène-5-diol-1,3 de formules A et B :

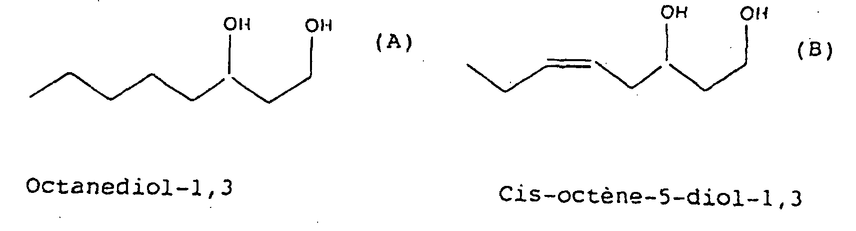

Le premier de ces diols-1,3 a d'abord été identifié dans un jus de pomme commercial (Brûlé et al., 1973) et trouvé en quantités très variables dans différentes variétés de pomme (Pyysalo et al., 1980), puis accompagné d'un homologue insaturé : le cis-octène-5-diol-1,3, présent en quantité beaucoup plus faible (Yajima et al., 1984). De plus, ces β-glycols dont la configuration absolue est R (Schwab et al., 1989) sont présents en quantité non négligeable sous forme liée glycosilée (Dettweiler et al., 1990 ; Schwab et al., 1989 ; Berger et al., 1988).

Kulesza, J. and Druri, M., *(Riechstoffe, Aromen, Körperpflegemittel*, **19**, 4, (1969), 157) ont publié un procédé de synthèse du 4-pentyldiol-1,3, décrit dans le contexte des molécules volatiles aromatiques employées dans la parfumerie. Le procédé décrit par Kulesza pour la synthèse du 4-pentyldiol-1,3 est basé sur de la chimie de laboratoire est utilise l'octène comme matière de départ.

La demanderesse a mis en évidence que ces diols-1,3 étaient des dérivés essentiels à la synthèse de composés de structure 1,3-dioxane présentant des propriétés aromatiques intéressantes, que la demanderesse a identifiés à l'état naturel dans des extraits de pomme ou de produits dérivés de la pomme. Néanmoins, l'assez faible quantité de diols-1,3 présents dans les pommes rendait la synthèse des composés de structure 1,3-dioxane peu intéressante sur le plan industriel.

C'est pourquoi la présente invention concerne un procédé de biosynthèse de diols-1,3 naturels de formule générale I : 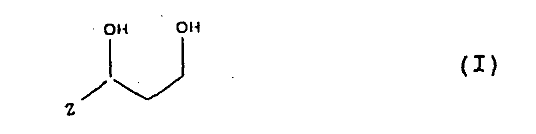 dans laquelle
Z est le groupement pentyle ou pent-2-ényle (Z) ou (E), de préférence (Z), qui consiste en la conversion d'un précurseur naturel desdits diols-1,3 à l'aide d'un complexe enzymatique et séparation éventuelle des diols obtenus.

En effet, on a pu mettre en évidence le fait que l'utilisation d'un précurseur naturel des diols-1,3 en cause, à savoir notamment un acide gras insaturé tel que l'acide linoléïque, permettait, sous l'action d'un complexe enzymatique, d'obtenir les diols mentionnés précédemment.

De façon préférée, le complexe enzymatique provient de la pomme, soit par extraction et purification, mais plus simplement en ajoutant l'acide linoléïque à un broyat de pommes et en laissant agir le système enzymatique qui s'y trouve.

Comme cela sera démontré dans ce qui va suivre, les proportions du broyat de pommes et du précurseur, notamment l'acide linoléïque, peuvent varier de façon assez large, en particulier la quantité de précurseur peut atteindre 4% du poids de pommes mis en oeuvre, bien que de préférence on utilise entre 1 et 3% du poids de pommes.

Le temps d'incubation peut dépendre du type de pomme utilisé, néanmoins l'incubation durera de 15 minutes à 6 heures, et de préférence de 3 heures à 5 heures.

Les études complémentaires ont montré que, parmi les broyats de pommes particulièrement intéressants, il fallait citer les broyats de plusieurs variétés de pommes à cidre.

Les essais réalisés et qui sont décrits dans les exemples montrent qu'en rajoutant de l'acide linoléïque il est possible de multiplier par trois ou quatre la quantité de diol-1,3 présente dans le fruit.

Les autres conditions de la biosynthèse peuvent être déterminées expérimentalement, on préférera mettre en oeuvre le procédé à une température comprise entre 15 et 25°C, bien que, dans certains cas, il soit possible d'utiliser des températures différentes.

Pour des raisons de prix, il n'est pas particulièrement intéressant de séparer le système enzymatique actif, néanmoins cela est possible en mettant en oeuvre les technologies bien connues en la matière.

Enfin, il n'est pas nécessaire d'avoir recours à des additifs particuliers, même si, dans certains cas, il est possible d'envisager des additifs, par exemple pour stopper la réaction enzymatique si cela est nécessaire.

Les diols-1,3 obtenus peuvent être séparés par toute méthode connue pour séparer ce type de diol, notamment. par des systèmes chromatographiques ou autre méthode de purification, sur résine par exemple.

Comme cela a été indiqué précédemment, ces produits sont particulièrement intéressants pour la synthèse de composés de structure 1,3-dioxane naturels, jusqu'à présent inconnus et identifiés pour la première fois par la demanderesse à l'état naturel dans des broyats de pommes et des extraits de produits dérivés de la pomme. Lesdits composés de structure 1,3-dioxane de formule générale II : 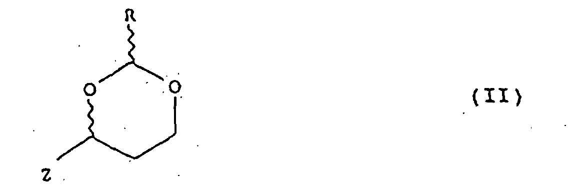 dans laquelle
Z est le groupement pentyle ou pent-2-ényle (Z) et
R est un groupement hydrocarboné, de préférence alkyle en C₁ à C₆,
sont obtenus par acétalisation spontanée des dérivés de diol-1,3 naturels de formule générale I correspondants, préparés par le procédé décrit précédemment, par un ou plusieurs aldéhydes naturels de formule :

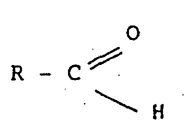

Le procédé d'acétalisation spontanée est mis en oeuvre de préférence avec un agent desséchant tel que la silice anhydre.

Pour réaliser cette acétalisation, on utilisera de préférence des aldéhydes naturels, notamment des aldéhydes aliphatiques en C₁ à C₆ ou aromatiques, c'est-à-dire dans lesquels le radical R est notamment un radical alkyle en C₁ à C₆ et/ou aromatique, notamment le radical phényle, et de préférence les aldéhydes en question seront choisis parmi l'éthanal, le propanal, le butanal et l'héxanal.

Les diols-1,3 naturels seront mis en présence d'une quantité stoechiométrique d'aldéhyde.

La réaction est conduite pendant une durée de 12 heures à 3 jours, de préférence 24 à 48 heures, à une température de l'ordre de 10 à 40°C.

Les composés de structure 1,3-dioxane naturels obtenus sont purifiés par distillation ou chromatographie sur colonne de silice et peuvent être analysés par chromatographie en phase gazeuse. Lorsqu'ils comportent des stéréoisomères, ceux-ci peuvent être séparés par toute méthode appropriée, notamment par chromatographie en phase gazeuse et notamment sur colonne chirale.

Comme cela sera indiqué dans ce qui suit, les composés de structure 1,3-dioxane présentent des notes intéressantes sur le plan aromatique.

C'est pourquoi, la présente invention concerne également des compositions contenant ces dioxanes et plus particulièrement les dérivés ci-après :

### Structure des 1,3-dioxanes formées à partir des diols-1,3 (composés A et B) et des aldéhydes éthanal, propanal, butanal, hexanal :

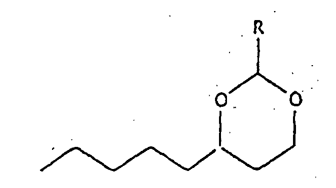

### Dérivés 4-pentyl-1,3-dioxane :

1: R = méthyl : 2-méthyl-4-pentyl-1,3-dioxane
2: R = éthyl : 2-éthyl-4-pentyl-1,3-dioxane
3: R = propyl : 2-propyl-4-pentyl-1,3-dioxane
4: R = pentyl : 2-pentyl-4-pentyl-1,3-dioxane

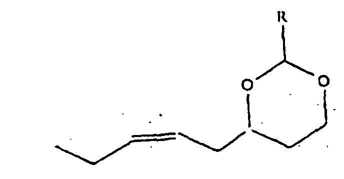

### Dérivés [4-(Z)-pent-2-ényl]-1,3-dioxane :

5 : R = méthyl.: 2-méthyl-[4-(Z)-pent-2-ényl]-1,3-dioxane
6 : R = éthyl : 2-éthyl-[4-(Z)-pent-2-ényl]-1,3-dioxane
7 : R = propyl : 2-propyl-[4-(Z)-gent-2-ényl]-1,3-dioxane
8 : R = pentyl : 2-pentyl-[4-(Z)-pent-2-ényl]-1,3-dioxane.

Les composés de structure 1,3-dioxane naturels ainsi obtenus possèdent des caractéristiques analytiques permettant de mettre en évidence leur origine naturelle ou synthétique.

L'analyse par chromatograpie en phase gazeuse sur colonne chirale des diols-1,3 saturés et insaturés ainsi que des 1,3-dioxanes (composés 1 à 8), dont ils sont les précurseurs, permet de différencier les produits d'origine naturelle des produits obtenus par voie de synthèse chimique.

Le procédé de préparation des composés de structure 1,3-dioxane naturels selon l'invention permet l'accès à une nouvelle classe de molécules aromatisantes possédant des notes vertes, fraîches et fruitées. La présente invention se rapporte également à toute composition aromatique ou de parfumerie contenant ces composés de structure 1,3-dioxane naturels.

Les exemples ci-après permettront de mettre en évidence d'autres avantages et caractéristiques de la présente invention.

### Exemple 1 : Détermination de la teneur en octanediol-1,3 et en cis-octène-5-diol-1,3 dans la pomme

100 g de pommes à cidre sont broyés en présence de 100 ml d'eau. Le broyat est ensuite extrait au dichlorométhane après addition d'un standard interne (undécanoate de méthyle). L'extrait est ensuite concentré jusqu'à 1 ml et analysé en chromatographie en phase gazeuse.

Les conditions d'analyse par chromatographie en phase gazeuse sont les suivantes :
- colonne FFAP (Hewlett Packard) ; 60m ; diamètre interne : 0,32 mm ; épaisseur du film ; 0,53µm ;
- colonne chirale ;
- chromatographe de type Hewlett Packard 6890 ;
- programmation de température et débits : isotherme à 60°C, 3 mn ; 3°C/mn jusqu'à 120°C ; 2°C/mn jusqu'à 220°C ; isotherme à 220°C, 60 mn ; gaz vecteur : hélium à 2 ml/mn maintenu constant par un contrôleur électronique de pression ; détecteur FID : hydrogène : 30 ml/mn.

Les teneurs en diols-1,3 de formules A et B varient beaucoup d'une variété de pomme à l'autre. C'est pourquoi, la demanderesse a recherché quelles variétés de pomme étaient les plus riches en composés A et B afin d'optimiser le procédé de préparation selon l'invention.

La teneur en diols-1,3 libres et liés sous forme de glucosides de différentes variétés de pomme a été déterminée en ajoutant 5% en poids de préparation enzymatique commerciale Rohapect B1L (Rhom), Novozym (Novo) ou PLM (Grindsted) par rapport à la quantité de pommes mise en oeuvre. Cette préparation enzymatique commerciale contient à la fois une activité pectinase et une activité glucosidase. L'homogénat enzymé est maintenu 24 heures à 30°C sous agitation avant extraction.

Des quantités importantes en ces diols-1,3 ont été trouvées, notamment dans certaines variétés de pomme à cidre, ce qui n'était pas rapporté dans la littérature jusqu'à présent.

La teneur en diols-1,3 dans deux variétés de pommes à cidre a été déterminée à différents stades de maturité du fruit, montrant ainsi que la quantité de diols-1,3 augmente considérablement lorsque le fruit mûrit. Conformément à ce qui était déjà connu, et comme le montre l'étude effectuée sur ces deux variétés de pomme à cidre (tableau 1), une partie importante des deux diols-1,3 se trouve dans le fruit à l'état non volatil, liée sous forme de glucosides. L'étude est faite à partir de fruits récoltés au mois d'octobre, analysés puis stockés et prélevés à un mois d'intervalle, au cours des mois de novembre et décembre (stade 1, 2 et 3) :

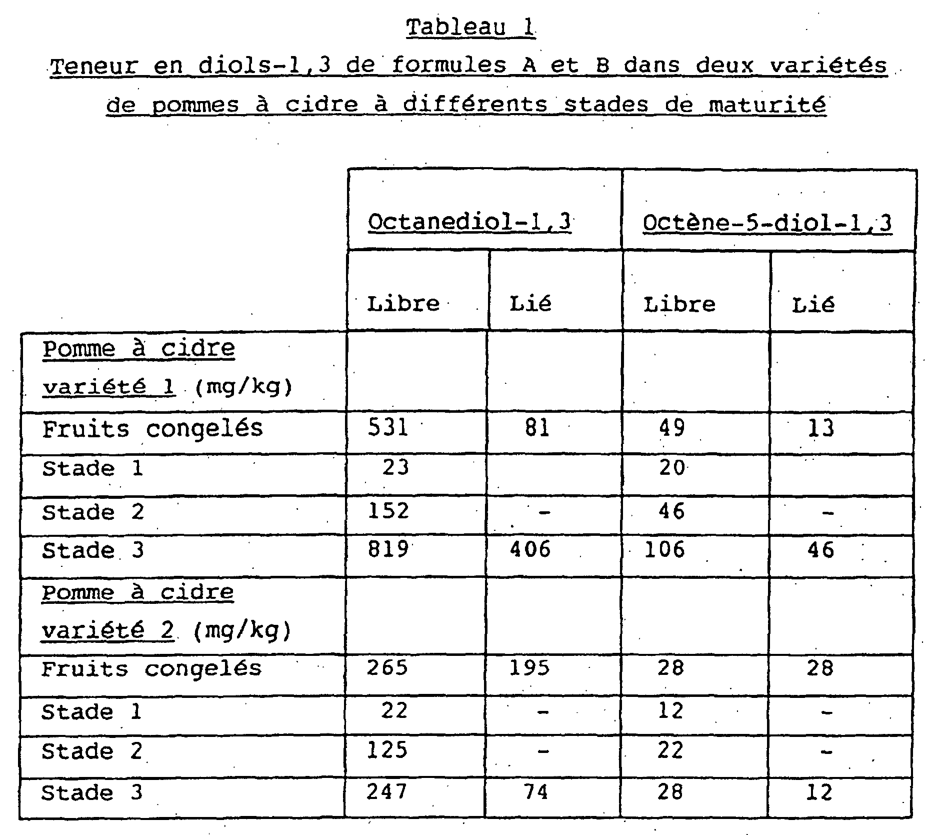

### Exemple 2 : Stimulation de l'activité enzymatique en vue de la production des diols-1,3 naturels

L'activité des systèmes enzymatiques impliqués dans la biosynthèse des diols-1,3 de formules A et B dépend à la fois de la variété de pommes mais aussi de la quantité de précurseur naturel disponible.

En effet, comme le montrent les résultats obtenus dans le tableau 2, seules les pommes à cidre possèdent une activité enzymatique suffisante pour produire des quantités importantes de diols-1,3, en présence d'acide linoléïque comme précurseur.

Par contre, trois variétés de pommes de table étudiées ont une activité enzymatique très faible ou nulle ne permettant pas la biosynthèse des deux diols-1,3 de formules A et B à partir de l'acide linoléïque.

**Tableau 2**

| Production des deux diols-1,3 de formules A et B, selon la variété de pomme, en présence d'acide linoléïque comme précurseur naturel | | |
|---|---|---|
| Variété | Octanediol-1,3 (g/kg de pomme) | Octène-5-diol-1,3 (g/kg de pomme) |
| Pomme à cidre variété 1 | 5,10 | 0,150 |
| Pomme à cidre variété 2 | 2,50 | 0,037 |
| Pomme de tablé variété 1 | 0,020 | 0,008 |
| Pomme de table variété 2 | 0 | 0 |
| Pomme de table variété 3 | 0,006 | 0,003 |

L'activité des sytèmes enzymatiques dépend également de la quantité de précurseur naturel disponible.

L'activité des systèmes enzymatiques peut donc être stimulée par addition de leur précurseur naturel, en l'occurrence d'un précurseur acide gras.

Un des acides gras majeur de la pomme étant l'acide linoléïque, celui-ci a été, dans un premier temps, ajouté à un broyat d'une variété de pommes à cidre en quantité variable.

100 g de pommes à cidre de la variété 1 sont broyés en présence de 150 ml d'eau et de 2 g d'acide linoléïque pur ou sous forme d'hydrolysat d'huile de lin. Après 4 heures d'incubation à température ambiante et sous agitation, l'homogénat est extrait au solvant après addition d'un standard interne comme dans l'exemple 1.

La quantité optimale d'acide linoléïque nécessaire à la formation des diols-1,3 de formules A et B a été ainsi déterminée par mesure de la quantité de diols-1,3 formés dans des homogénats de pommes à cidre contenant des quantités croissantes d'acide gras.

La teneur des deux diols-1,3 est déterminée en chromatographie en phase gazeuse (GC) après extraction et concentration de l'extrait. Les résultats obtenus sont indiqués dans le tableau 3.

**Tableau 3**

| Détermination de la quantité optimum d'acide linoléïque nécessaire à la biosynthèse des diols-1,3 naturels de formules A et B dans une pomme à cidre de la variété 1 | | |
|---|---|---|
| Acide linoléïque ajouté (g/kg de pomme) | Octanediol-1,3 (g/kg de pomme) | Octène-5-diol-1,3 (g/kg de pomme) |
| O (témoin) | 1,05 | 0,033 |
| 10 | 2,40 | 0,094 |
| 15 | 3,80 | 0,124 |
| 20 | 4,17 | 0,127 |
| 25 | 3,30 | 0,098 |
| 30 | 2,70 | 0,092 |
| 40 | 2,50 | 0,077 |

Le maximum de production des deux diols-1,3 est atteint pour une quantité d'acide linoléïque de 2% par rapport au poids de pommes à cidre de la variété 1 mis en oeuvre. L'ajout de quantités plus importantes de précurseur acide gras provoque une sensible diminution de la quantité de diols-1,3 produite, très probablement par inhibition des systèmes enzymatiques par le substrat.

La quantité optimale d'acide linoléïque nécessaire à la formation des diols-1,3 étant de 2% par rapport au poids de pommes à cidre de la variété 1 mis en oeuvre, le temps d'incubation optimal dans un homogénat de pommes a ensuite été déterminé (tableau 4) en utilisant cette quantité de précurseur.

**Tableau 4**

| Cinétique de formation des deux diols-1,3 naturels de formules A et B dans un broyat de pommes cidre de la variété 1 | | |
|---|---|---|
| Temps | Octanediol-1,3 (g/kg de pomme) | Octène-5-diol-1,3 (g/kg de pomme |
| T0 (témoin) | 1,13 | 0,033 |
| T + 5 min | 1,60 | 0,044 |
| T + 10 min | 2,15 | 0,058 |
| T + 15 min | 3,30 | 0,096 |
| T + 30 min | 4,00 | 0,130 |
| T + 60 min | 4,10 | 0,135 |
| T + 150 min | 4,45 | 0,140 |
| T + 240 min | 4,75 | 0,160 |
| T + 360 min | 4,75 | 0,140 |

De la cinétique ci-dessus, il apparaît que la quantité maximale des diols-1,3 naturels de formules A et B est obtenue après 4 heures d'incubation en présence de l'acide linoléïque comme précurseur. La teneur en octanediol-1,3 est alors augmentée d'environ un facteur trois. De même, la teneur en octène-5-diol-1,3 augmente dans les mêmes proportions, mettant ainsi en évidence que l'acide linoléïque est à l'origine de ces composés par une voie métabolique, pour le moment inconnue, mais impliquant probablement des enzymes du type lipoxygénase, spécifiques de la pomme.

### Exemple 3 : Préparation des composés de structure 1,3-dioxane naturels (composés 1 à 8)

L'ensemble de ces composés (1 à 8), importants sur le plan sensoriel, sont obtenus à l'état naturel par réaction spontanée des aldéhydes correspondants et des diols-1,3 homologues isolés de la pomme selon la méthode suivante :

Les diols-1,3 sont mis en présence d'une quantité stoechiométrique d'aldéhyde (éthanal, propanal, butanal ou hexanal) et de silice anhyde sous agitation pendant 48 heures à température ambiante.

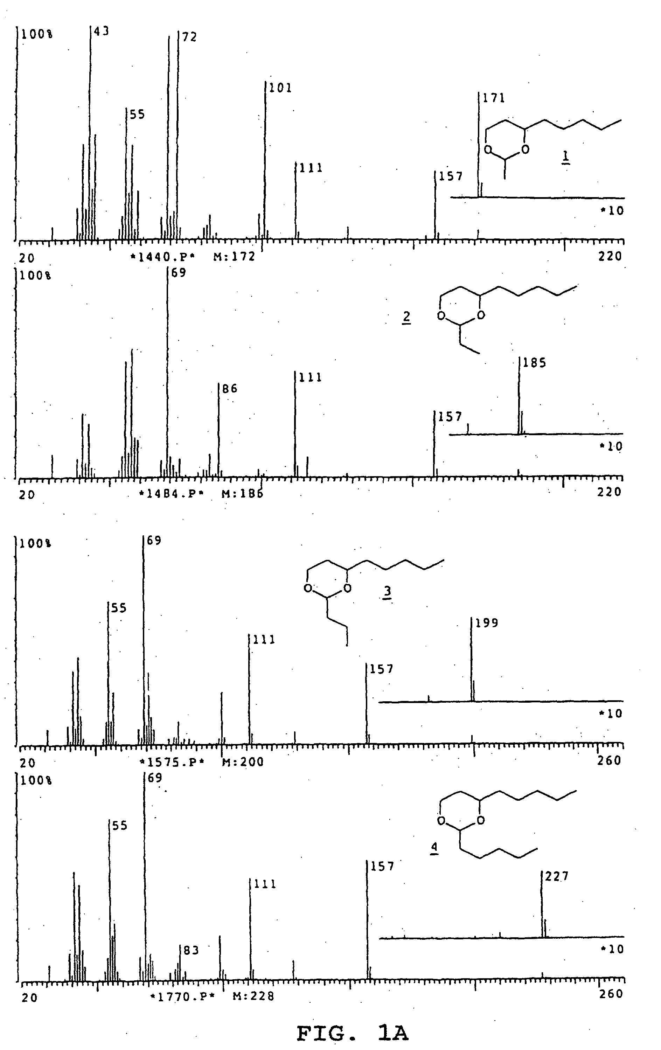

Le milieu réactionnel est ensuite analysé par CG et les composés de structure 1,3-dioxane sont dosés. Les rendements obtenus pour les composés 1 à 4 sont les suivants (tableau 5). Les spectres de masse des composés 1 à 4 sont présentés figure 1a.

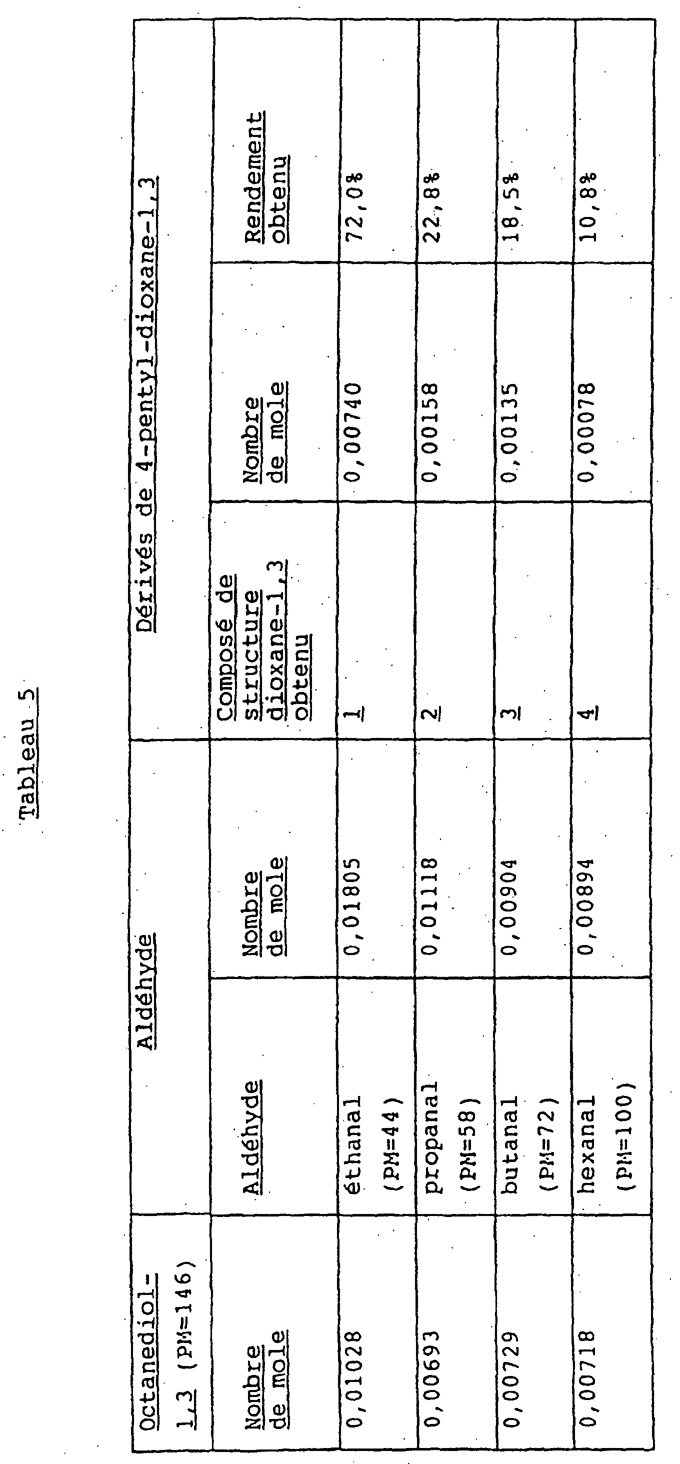

Les rendements obtenus pour les composés 5 à 8 n'ont pu être calculés.

La synthèse des composés de structure 1,3-dioxane insaturés (5 à 8) peut être effectuée par voie chimique à partir de l'octène-5-diol-1,3 préalablement isolé d'un homogénat de pommes à cidre, traité selon le procédé de l'invention, et des aldéhydes naturels, éthanal, propanal, butanal et hexanal en présence d'une quantité catalytique d'acide.

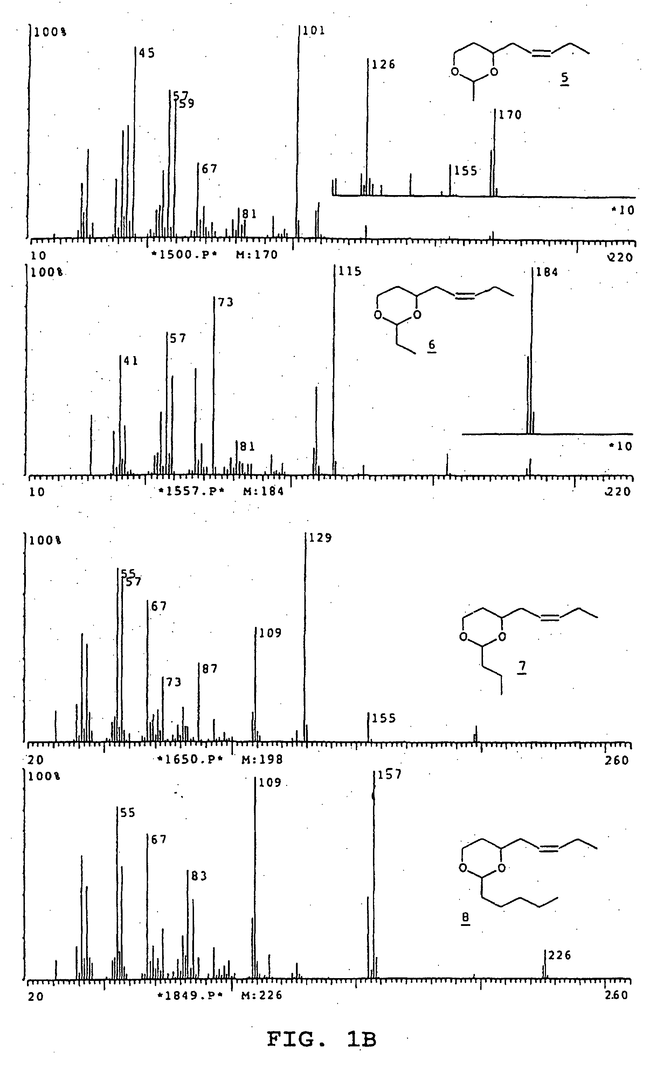

Ainsi, et en tant que références analytiques, les spectres de masse des quatre composés de structure 1,3-dioxane possédant une chaîne latérale insaturée (composés 5 à 8), obtenus par synthèse chimique sont présentés figure 1b.

Les conditions d'analyse par couplage GC/MS utilisent les mêmes conditions chromatographiques décrites dans l'exemple 1 et un spectromètre de masse à secteur magnétique de type Profile (Kratos) réglé en mode EI ; énergie d'ionisation : 70 ev, vide : 2,10-5 torr ; température de source : 150°C ; température de l'interface: 240°C.

Les composés obtenus 1 à 8 peuvent être analysés en GC/Olfactométrie, les caractéristiques sensorielles de chacun d'eux peuvent ainsi être évaluées (tableau 6).

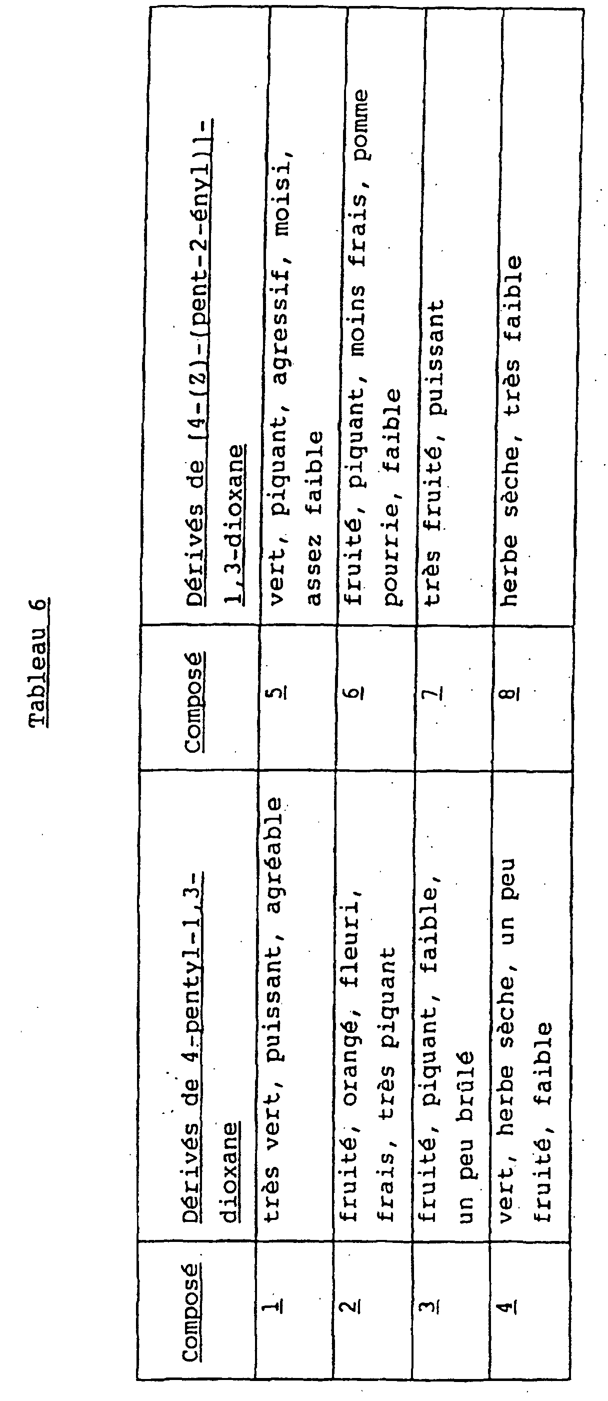

### Exemple 4 : Isolation et identification des énantiomères des composés de structure 1,3-dioxane naturels

L'octanediol-1,3 et l'octène-5-diol-1,3 possèdent un carbone asymétrique qui conduit à la possibilité d'isomérie optique. Les énantiomères de ces composés, lorsqu'ils sont présents, peuvent être séparés par chromatographie en phase gazeuse sur colonne chirale permettant ainsi la mise en évidence de l'origine des diols-1,3, les composés obtenus par synthèse étant racémiques alors que les diols extraits de la pomme sont énantiomériquement purs.

Lors de la réaction entre les diols-1,3 et les aldéhydes de la pomme (éthanal, propanal, butanal et hexanal) qui conduit à la formation des dioxanes 1 à 8, un carbone asymétrique supplémentaire est introduit, d'où la présence de diastéréoisomères séparables en chromatographie en phase gazeuse sur colonne normale. Un des diastéréoisomères est largement majoritaire. L'analyse sur colonne chirale de ces composés permet la séparation des énantiomères de chacun des diastéréoisomères. Cette analyse permet'la différenciation des composés 1 à 8 en fonction de leur origine naturelle ou de synthèse.

La stéréoisomérie des composés de structure dioxane-1,3. (composés 1 à 8) est influencée par la longueur de lachaîne latérale portée par le noyau 1,3-dioxane.

Les conditions d'analyse par chromatographie en phase gazeuse sont les mêmes que dans l'exemple 1.

## Revendications

1. Procédé de biosynthèse des dérivés de diol-1,3 naturels de formule générale I 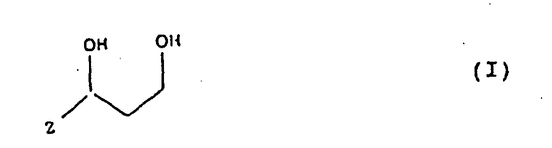 dans laquelle
Z est le groupement pentyle ou pent-2-ényle (Z) ou (E), de préférence (Z), qui consiste en la conversion d'un précurseur naturel desdits diols à l'aide d'un complexe enzymatique, pouvant tous deux provenir de la pomme.

2. Procédé selon la revendication 1, **caractérisé en ce que** le précurseur naturel est un acide gras insaturé.

3. Procédé selon la revendication 2, **caractérisé en ce que** l'acide gras insaturé est l'acide linoléïque.

4. Procédé selon la revendication 3, **caractérisé en ce que** l'acide linoléïque provient de la pomme ou d'un hydrolysat d'huile végétale.

5. Procédé selon l'une quelconque des revendications 1 et 2, **caractérisé en ce que** le complexe enzymatique provient de la pomme.

6. Procédé selon la revendication 5, **caractérisé en ce que** le complexe enzymatique est un broyat de pommes.

7. Procédé selon l'une quelconque des revendications 1 à 6, **caractérisé en ce que** l'acide linoleïque est ajouté à un broyat de pommes dans des proportions pouvant varier jusqu'à 4% du poids de pomme mis en oeuvre.

8. Procédé selon la revendication 7, **caractérisé en ce que** la proportion d'acide linoleïque ajouté au broyat de pommes est comprise entre 1 et 3% du poids de pommes mis en oeuvre.

9. Procédé selon l'une quelconque des revendications 1 à 8, **caractérisé en ce que** le temps d'incubation du mélange broyat de pommes/acide linoléïque est compris entre 15 minutes et 6 heures.

10. Procédé selon la revendication 9, **caractérisé en ce que** le temps d'incubation du mélange broyat de pommes/acide linoléïque est compris entre 3 et 5 heures.

11. Procédé selon l'une quelconque des revendications 1 à 10, **caractérisé en ce que** les pommes utilisées sont des pommes à cidre.

12. Procédé selon l'une des revendications 1 a 11, **caractérisé en ce que** les dérivés de diol-1,3 naturels de formule générale I sont isolés du broyat de pommes par absorption sur résine.

13. Procédé de préparation des composés de structure 1,3-dioxane naturels de formule générale II 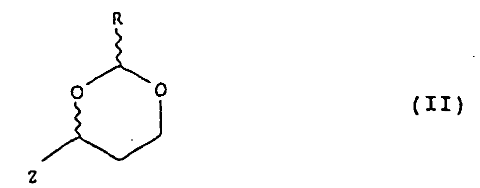 dans laquelle
Z est le groupement pentyle ou pent-2-ényle (Z) et
R est un groupement hydrocarboné,
par acétalisation spontanée des dérivés de diol-1,3 naturels de formule générale I correspondants, préparés par le procédé selon les revendications 1 à 9, par un ou plusieurs aldéhydes naturels de formule :

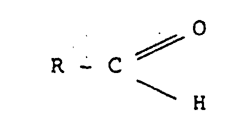

14. Procédé selon la revendication 13, **caractérisé en ce que** les dérivés de diol-1,3 naturels de formule générale I sont mis en présence d'une quantité stoechiométrique d'aldéhyde(s) naturel(s) et d'un agent desséchant, de préférence la silice anhydre.

15. Procédé selon l'une quelconque des revendications 13 et 14, **caractérisé en ce que** le(s) aldehyde(s) naturel(s) est (sont) de préférence aliphatique(s) en C₁-C₆ ou aromatique(s).

16. Procédé selon la revendication 15, **caractérisé en ce que** le(s) aldéhyde(s) naturel(s) est (sont) choisi(s) parmi l'éthanal, le propanal, le butanal et l'héxanal.

17. Procédé selon l'une quelconque des revendications 13 à 16, **caractérisé en ce que** l'acétalisation est conduite sous agitation pendant environ 24 à 48 heures à une température comprise entre 10 et 40°C.

18. Procédé selon l'une quelconque des revendications 13 à 17, **caractérisé en ce que** le(s) composé(s) de structure 1,3-dioxane naturel(s) obtenu(s) de formule générale II est (sont) purifié(s) par distillation ou chromatographie sur colonne de silice.

19. Procédé selon la revendication 18, **caractérisé en ce que** chaque composé de structure 1,3-dioxane naturel de formule générale II isolé, obtenu sous forme d'un mélange de diastéréoisomères, est analysé par chromatographie en phase gazeuse sur colonne chirale afin de séparer ses énantiomères.

20. Compositions contenant les composés de structure 1,3-dioxane naturels de formule générale II obtenus à partir du procédé selon l'une des revendications 13 à 19.

## Claims

1. Method of biosynthesis of natural 1,3-diol derivatives of general formula I 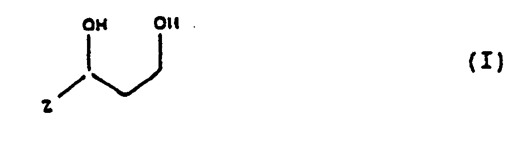 in which
Z is the pentyl group or the (Z)- or (E)-pent-2-enyl group, preferably the (Z) group, which consists in the conversion of a natural precursor of said diols with the aid of an enzymatic complex, both of which may originate from the apple.

2. Method according to Claim 1, **characterized in that** the natural precursor is an unsaturated fatty acid.

3. Method according to Claim 2, **characterized in that** the unsaturated fatty acid is linoleic acid.

4. Method according to Claim 3, **characterized in that** the linoleic acid originates from the apple or from a vegetable oil hydrolysate.

5. Method according to either of Claims 1 and 2, **characterized in that** the enzymatic complex originates from the apple.

6. Method according to Claim 5, **characterized in that** the enzymatic complex is an apple mash.

7. Method according to any one of Claims 1 to 6, **characterized in that** the linoleic acid is added to an apple mash in variable proportions of up to 4% of the weight of apple employed.

8. Method according to Claim 7, **characterized in that** the proportion of linoleic acid added to the apple mash is between 1 and 3% of the weight of apples employed.

9. Method according to any one of Claims 1 to 8, **characterized in that** the incubation time of the apple mash/linoleic acid mixture is between 15 minutes and 6 hours.

10. Method according to Claim 9, **characterized in that** the incubation time of the apple mash/linoleic acid mixture is between 3 and 5 hours.

11. Method according to any one of Claims 1 to 10, **characterized in that** the apples employed are cider apples.

12. Method according to one of Claims 1 to 11, **characterized in that** the natural 1,3-diol derivatives of general formula I are isolated from the apple mash by absorption on resin.

13. Method of preparing natural compounds having a 1,3-dioxane structure, of general formula II 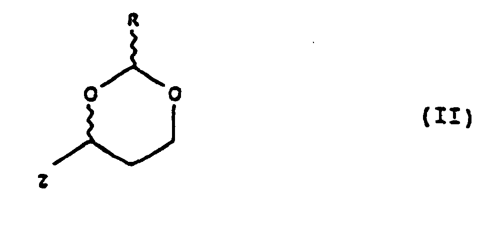 in which
Z is the pentyl or (Z)-pent-2-enyl group and
R is a hydrocarbon group,
by spontaneous acetalization of the corresponding natural 1,3-diol derivatives of general formula I, prepared by the method according to one of Claims 1 to 9, with one or more natural aldehydes of formula

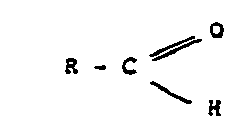

14. Method according to Claim 13, **characterized in that** the natural 1,3-diol derivatives of general formula I are placed in the presence of a stoichiometric amount of natural aldehyde(s) and a desiccant, preferably anhydrous silica.

15. Method according to either of Claims 13 and 14, **characterized in that** the natural aldehyde(s) is (are) preferably C₁-C₆ aliphatic(s) or aromatic(s).

16. Method according to Claim 15, **characterized in that** the natural aldehyde(s) is (are) selected from ethanal, propanal, butanal and hexanal.

17. Method according to any one of Claims 13 to 16, **characterized in that** the acetalization is conducted with stirring for from approximately 24 to 48 hours at a temperature of between 10 and 40°C.

18. Method according to any one of Claims 13 to 17, **characterized in that** the natural compound(s) having a 1,3-dioxane structure that is (are) obtained, of general formula II, is (are) purified by distillation or chromatography on a silica column.

19. Method according to Claim 18, **characterized in that** each natural compound having a 1,3-dioxane structure that is isolated, of general formula II, obtained in the form of a mixture of diastereoisomers, is analyzed by gas chromatography on a chiral column in order to separate its enantiomers.

20. Compositions comprising the natural compounds having a 1,3-dioxane structure, of general formula II, obtained from the method according to one of Claims 13 to 19.

## Patentansprüche

1. Verfahren zur Biosynthese natürlicher 1,3-Diolderivate der allgemeinen Formel I 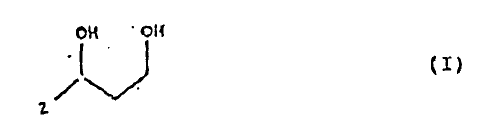 in der
Z eine Pentyl- oder Pent-2-enyl-Gruppe (Z) oder (E), bevorzugt (Z), ist, welches in der Umwandlung cines natürlichen Vorläufers der Diole mit Hilfe eines enzymatischen Komplexes, welche beide aus dem Apfel stammen können, besteht.

2. Verfahren gemäß Anspruch 1, **dadurch gekennzeichnet, dass** der natürliche Vorläufer eine ungesättigte Fettsäure ist.

3. Verfahren gemäß Anspruch 2, **dadurch gekennzeichnet, dass** die ungesättigte Fettsäure Linolsäure ist.

4. Verfahren gemäß Anspruch 3, **dadurch gekennzeichnet, dass** die Linolsäure aus dem Apfel oder dem Hydrolysat eines Pflanzenöls stammt.

5. Verfahren nach wenigstens einem der Ansprüche 1 und 2, **dadurch gekennzeichnet, dass** der enzymatische Komplex aus dem Apfel stammt.

6. Verfahren gemäß Anspruch 5, **dadurch gekennzeichnet, dass** der enzymatische Komplex ein Zerrieb von Äpfeln ist.

7. Verfahren nach wenigstens einem der Ansprüche 1 bis 6, **dadurch gekennzeichnet, dass** die Linolsäure einem Apfelzerrieb mit einem Verhältnis, welches bis zu 4 Gew.-% der eingesetzten Äpfel betragen kann, zugefügt wird.

8. Verfahren gemäß Anspruch 7, **dadurch gekennzeichnet**, dase das Verhältnis der Linolsäure, welche dem Apfelzerrieb zugefügt wird, zwischen 1, und 3 Gew.-% der eingesetzten Äpfel beträgt.

9. Verfahren nach wenigstens einem der Ansprüche 1 bis 8, **dadurch gekennzeichnet, dass** die Inkubationszeit der Mischung Apfelzerrieb/Linolsäure zwischen 15 Minuten und 6 Stunden beträgt.

10. Verfahren gemäß Anspruch 9, **dadurch gekennzeichnet, dass** die Inkubationszeit der Mischung Apfelzerrieb/Linolsäure zwischen 3 und 5 Stunden beträgt.

11. Verfahren nach wenigstens einem der Ansprüche 1 bis 10, **dadurch gekennzeichnet, dass** die verwendeten Apfel Cidre-Äpfel sind.

12. Verfahren nach wenigstens einem der Ansprüche 1 bis 11, **dadurch gekennzeichnet, dass** die natürlichen 1,3-Diolderivate der allgemeinen Formel I aus dem Apfelzerrieb durch Absorption auf einem Harz isoliert werden.

13. Verfahren zur Herstellung von natürlichen Verbindungen mit 1,3-Dioxanstruktur der allgemeinen Formel II 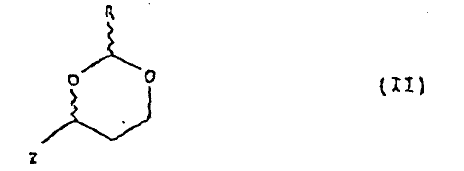 in der
Z eine Pentyl- oder Pent-2-enyl-Gruppe (Z) und R cine Kohlenwasserstoffgruppe sind, durch spontane Acetalisierung der entsprechenden natürlichen 1,3-Diolderivate der allgemeinen Formel I, welche durch das Verfahren gemäß den Ansprüchen 1 bis 9 hergestellt wurden, durch ein oder mehrere natürliche Aldehyde der Formel

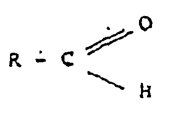

14. Verfahren gemäß Anspruch 13, **dadurch gekennzeichnet, dass** die natürlichen 1,3-Diolderivatc der allgemeinen Formel I mit einer stöchiometrischen Menge an natürlichem (natürlichen) Aldehyd(cn) und einem Trockenmittel, bevorzugt wasserfreiem Kieselsäureanhydrid, zusammengebracht werden.

15. Verfahren nach wenigstens einem der Ansprüche 13 und 14, **dadurch gekennzeichnet, dass** das (die) natürliche(n) Aldehyd(e) bevorzugt aliphatisch mit C₁ bis C₆ oder aromatisch ist. (sind).

16. Verfahren gemäß Anspruch 15, **dadurch gekennzeichnet, dass** das (die) natürliche(n) Aldehyd(e) Ethanal, Propanal, Butanal und/oder Hexanal ist (sind).

17. Verfahren nach wenigstens einem der Ansprüche 13 bis 16, **dadurch gekennzeichnet, dass** die Acetalisierung unter Rühren über einen Zeitraum von etwa 24 bis 48 Stunden und einer Temperatur zwischen 10 und 40 °C durchgeführt wird.

18. Verfahren nach wenigstens einem der Ansprüche 13 bis 17, **dadurch gekennzeichnet, dass** die erhaltene(n) natürliche(n) Verbindung(en) mit 1,3-Dioxanstruktur der allgemeinen Formel II durch Destillation oder Chromatografie auf einer Kieselerdesäule gereinigt wird (werden).

19. Verfahren gemäß Anspruch 18, **dadurch gekennzeichnet, dass** jede isolierte natürliche Verbindung mit 1,3-Dioxanstruktur der allgemeinen Formel II, welche in Form einer Mischung von Diastereoisomeren erhalten wird, durch Gasphasen-Chromatographie auf einer chiralen Säule analysiert wird, um die Enantiomeren zu trennen.

20. Zusammensetzungen, welche die natürlichen Verbindungen mit 1,3-Dioxanstruktur der allgemeinen Formel II enthalten, welche durch das Verfahren gemäß einem der Ansprüche 13 bis 19 erhalten wurden.
